# EUROPEAN PATENT APPLICATION

(11) **EP 0 616 817 A1**
(43) Date of publication of application: **28.09.1994**
(21) Application number: 93309921.0
(22) Date of filing: 09.12.1993
(51) Int. Cl.: A61M 25/00, A61M 25/10, A61M 29/02

(54) **Catheter and manifold assembly and method of manufacture thereof**

(30) Priority: 10.03.1993 US 28988
(71) Applicant: C.R. BARD, INC., Murray Hill New Jersey 07974 (US)
(72) Inventor: Bourne IV, George W., Groton, Massachusetts 01450 (US); McIntyre, Jon T., Lowell, Massachusetts 01854 (US); Mackey, John J., Pelham, Newhampshire 03076 (US); Sellers, James S., Haverhill, Massachusetts 01830 (US)
(74) Representative: Woodward, John Calvin

(57) **Abstract**

A manifold comprising a manifold body (20) having a longitudinal passage (22) and proximal and distal port openings (26,28). The distal port opening (28) is secured by molding to an inflation tube (18) that defines an inflation lumen. An obliquely extending inflation leg (30) has an inflation passage (32) extending from a balloon inflation port (34) at the end of the leg into communication with the inflation tube lumen (18). A plug cap (50) having a longitudinal through bore (54) is secured within the proximal port opening (26). An inner guidewire tube (16) is coaxially positioned within the outer tube (18) and extends into the longitudinal passage of the manifold body (20). The tube is secured by molding to the plug cap (50). A flexible polymer strain relief jacket (40) is molded over the distal portion of the manifold body (20). A portion of the strain relief jacket (40) tapers inward and extends over the outer tube (18) distally of the distal port opening (28) to allow bending and flexing of the tubes adjacent the manifold distal end without kinking of the tubes. The strain relief jacket (40) is preferably molded over a majority of the surface of the manifold body (20) and a majority of the surface of the inflation leg (30).

## Description

This invention relates to a catheter manifold assembly and more particularly, to an overmolded angioplasty catheter manifold assembly having a flexible polymer strain relief molded over the distal portion of a manifold body through which inflation and guidewire tubes extend so as to allow bending and flexing of the tubes adjacent the manifold distal end without kinking of the tubes.

In angioplasty procedures, a catheter having an inflatable balloon is guided through arteries to a stenosis. In most conventional balloon catheters used in angioplasty, the inflatable balloon is positioned at the distal end of the catheter. A manifold, sometimes referred to as a "bifurcate" or "trifurcate", is positioned at the proximal end of the catheter. A cardiologist uses the manifold for manipulating the catheter, receiving a guidewire, connecting the balloon to a controllable source of inflation pressure, connecting the manifold to luer fittings, and controlling inflation of the balloon via inflation-deflation devices connected to the manifold.

In a typical balloon catheter, an outer inflation tube is positioned coaxially around an inner guidewire tube. Both tubes extend substantially the length of the catheter, defining an annular inflation lumen between the two tubes and a guidewire lumen within the inner tube. At their proximal end, the tubes connect to a manifold body having an obliquely extending inflation leg. The inflation leg has an inflation passage extending from a balloon inflation port at the proximal end of the leg into communication with the inflation tube lumen. In many designs, the guidewire is inserted through the proximal port opening of the manifold, into the inner tube, and through the balloon positioned at the distal end of the catheter. A conventional inflation-deflation device connects to the inflation leg.

In one known prior art manifold, the manifold is molded out of a Lexan body having metal luer locks held in a skived tube with adhesive. In use, the metal luers tended to crack the Lexan body when it was torqued, such as when connected to inflation devices, Y-bodies, or luer locks. Also, the adhesives used for securing the tubes and manifold body together tended to weaken or break, resulting in "cross-talk", i.e. the undesirable fluid flow between the different lumens and tubes. In another known manifold, two "legs" are heat-sealed with a shrink tube to form a manifold housing. This structure, however, is complex to manufacture, does not provide adequate support to the tubes after exiting the manifold, and does not have a smooth, ergonomic "feel" when handled.

Additionally, many of the known prior art manifolds do not always prevent kinking of the catheter tubes adjacent the distal end of the manifold. The tubes often are formed of a flexible, polymeric material such as polyethylene, which easily kinks when a smooth transition from the manifold is not provided.

Some prior art manifolds, such as the manifolds disclosed in US Patent No. 4.838.269, add a strain relief sleeve into the distal opening of the manifold to add support to the tubes exiting the manifold. However, such a sleeve typically does not provide a smooth transition from the manifold onto the outer catheter tube. Additionally, such a strain relief sleeve does not enhance the ergonomics and "touch" of the overall surface of the manifold to assist manual manipulation and create a more comfortable "touch" for the cardiologist during the tense minutes of an angioplasty procedure.

Often the cardiologist spends tedious minutes manipulating the guidewire and handling the manifold. What has been lacking in known devices is a catheter manifold assembly that provides a soft "feel" which results in a comfortable fit in the palm of the user's hand, has non-slip characteristics, facilitates manipulation, especially at the distal end of the manifold, and provides tactile feedback to the user to assist locating the catheter within the patient.

It is therefore an object of the present invention to provide a catheter manifold assembly that adequately secures the inflation tube and guidewire tube to the manifold while providing a strain relief that allows bending and flexing of the catheter tubes without kinking.

It is a further object of the present invention to provide a catheter manifold assembly wherein the manifold is formed to have a soft, comfortable and slip-resistant feel in the user's hand, facilitate manipulation of the assembly at the manifold, and provide tactile feedback to the user to assist the user in locating the catheter within a patient.

It is another object of the present invention to provide a catheter manifold assembly wherein the inflation and guidewire tubes of the catheter are secured to the manifold body so as to impart resilience and strength to the distal end of the manifold through which the tubes exit.

It is still another object of the present invention to provide a catheter manifold assembly wherein the flexible, polymeric tubes forming the inflation and guidewire lumens of the catheter are molded to the manifold to provide a strong, secure thermoplastic bond between the catheter tubes and the manifold.

It is still another object of the present invention to provide a catheter manifold assembly that provides sufficient strength between the tubes and the manifold body to prevent leaks, or "cross-talk" between the different tubes and lumens.

It is still another object of the present invention to provide a method of manufacturing a manifold that is simple and cost effective, while providing strength between the various inflation and guidewire tubes and the manifold body.

According to one aspect of the invention there is provided a catheter manifold assembly characterised by a manifold body of unitary construction having a proximal end, a distal end, an outer surface, and a longitudinal through passage defining a distal port at said distal end, a proximal port at said proximal end and a manifold inner surface, an elongated first catheter element having a proximal portion disposed within said manifold longitudinal passage and bonded to the inner surface of said manifold body, there being a thermoplastic bond portion between said first catheter element and said manifold body, the first catheter element extending distally beyond the distal port of the manifold body and having a longitudinal lumen, an elongated second catheter elements disposed in said manifold body longitudinal passage and extending distally beyond the distal port of the manifold body, said second catheter element having a proximal end bonded to the inner surface of the manifold body, there being a thermoplastic bond portion between said manifold body and said second catheter element, and a flexible polymer tubular strain relief jacket molded around the outer surface of said manifold body and having a distal portion extending distally beyond the distal end of said manifold body, said distal portion of the strain relief jacket being molded around said first and second catheter elements.

The preferred catheter manifold assembly of the present invention virtually eliminates cross-talk between the tubes defining the inflation and guidewire lumens of the catheter. The assembly has a flexible strain relief jacket that 1) allows bending and flexing of the tubes adjacent to the manifold's distal end without kinking of the tubes, 2) provides for a soft, comfortable, slip-resistant touch to the manifold that provides tactile feedback to the user and 3) aids in securing the flexible catheter tubes to the manifold.

In one embodiment, the manifold has a manifold body with a longitudinal passage extending between proximal and distal port openings. The manifold may be molded to an inflation tube just proximal to and beginning at the distal port opening. An obliquely extending inflation leg on the manifold has an inflation passage extending from a balloon inflation port at the proximal end of the inflation leg into communication with the inflation tube lumen. Preferably, the manifold body is insert molded, e.g. "injection molded" over the inflation tube.

In a preferred embodiment, a guidewire tube extends into the longitudinal passage of the manifold body and is secured in an area adjacent to the proximal port opening of the manifold body. Preferably, the guidewire and inflation tubes are coaxial to each other, the guidewire tube being the inner tube. The inflation tube and guidewire tube may also be disposed in a side by side arrangement. A flexible polymer strain relief is molded over the distal portion of the manifold body. A portion of the strain relief is molded over the tubes distally of the distal port opening of the manifold body and tapers distally from the manifold body to the distal tip of the strain relief. Preferably, the distal portion of the strain relief includes means for enhancing its flexibility, thus facilitating bending and flexing of the tubes adjacent to the manifold's distal end without kinking of the tubes.

Preferably, a plug cap is received within the proximal end of the manifold body passage and ultrasonically welded to the proximal end of the manifold body. The plug cap has a throughbore and may be insert molded, e.g. "injection molded" over the inner tube in a separate manufacturing step.

The flexible polymer strain relief jacket is preferably overmolded onto the manifold body. The strain relief jacket is molded over a majority of the surface area of the manifold body and a majority of the surface area of the inflation leg. The soft, flexible strain relief jacket material and the concentric ribs on the distal end of the strain relief jacket enhance the ergonomic "touch" and slip resistance of the strain relief jacket. An enhanced "ergonomic touch" aids the cardiologist considerably during the tedious minutes of an angioplasty procedure, where slight finger slippage or an uncomfortable feeling during finger manipulation could deter the cardiologist's attention. In addition, lack of tactile feedback could impede proper catheter and/or guidewire placement in the patient. The ribs also increase the bendability or flexibility of the distal portion of the strain relief jacket. The strain relief jacket can be formed from any elastomeric material having a hardness ranging from 50 to 100 on the Shore A hardness scale. In one embodiment, the strain relief jacket is formed from a block copolymer, composed of polystyrene segments in a matrix of polybutadiene or polyisoprene, known conventionally under the trade name KRATON™ "G". The distal portion of the strain relief jacket may also be formed from an elastomeric foam material which enhances its flexibility. In addition, to increase the "feel" of the device, the distal portion of the strain relief jacket may be formed from a material having a comparatively high coefficient of friction such as a soft rubber.

The manifold body and plug cap may be formed of a polyelefin such as polyethylene, and preferably high density polyethylene having a dimensionally stable filler material such as microscopic glass spheres that preferably range from about 9 to about 13 microns in size. They can be approximately 11 microns average size. Preferably, the glass spheres are added about 17% to 23% by weight, averaging about 20% by weight. The percentage by weight of added glass spheres can range as low as 10% by weight to as high as 30% by weight. The glass filler strengthens the high density polyethylene resulting in better ultrasonic welding between the plug cap and manifold polyethylene body, as well as the enhanced bond created during injection molding between the tubes and respective manifold body or plug cap. Additionally, the glass filler provides strength to the manifold body and plug cap, thus imparting strength to the threads or other fastening means formed on the manifold.

In addition to a strength enhancing filler, the manifold body and plug cap may include a phosphorescent filler that provides the assembly with "glow-in-the-dark" characteristics.

It should be noted that while the subject assembly is described in the context of angioplasty devices the invention is not so limited. As an example, but not intended to be limiting, the subject assembly is applicable to porous balloon catheters which may be used to administer a fluid medicament to a blood vessel. In addition, while the invention is discussed in the context of "bifurcate" or "trifurcate" manifolds, it is contemplated that the invention may be incorporated into manifolds having one or more than three proximal ports. Other applications of the invention will be obvious to those skilled in the catheter art.

The present invention will now be described, by way of example, with reference to the accompanying drawings in which:
Figure 1 is an environmental view of the manifold of the present invention showing its use with an inflatable balloon;
Figure 1A is an enlarged sectional view of the catheter shaft showing inner and outer tubes;
Figure 2 is a sectional view of the manifold of the present invention taken along line 2-2 of Figure 3;
Figure 3 is an end view of the manifold of the present invention looking in the direction of arrow 3 of Figure 1;
Figure 4 is a block diagram illustrating the steps in forming the manifold of the present invention;
Figure 5 is a schematic representation of the formed manifold body after insert molding onto the outer inflation tube;
Figure 6 is a schematic representation of the manifold body after insert molding the strain relief onto the outer inflation tube and manifold body;
Figure 7 is a schematic representation showing the plug cap insert molded onto the inner guidewire tube;
Figure 8 is a schematic representation showing the plug cap and inner guidewire tube inserted through the proximal port opening of the manifold;
Figure 9 is a schematic representation showing ultrasonic welding of the plug cap to the manifold body;
Figure 10 is a cross-sectional view of another embodiment of the subject assembly wherein the catheter includes first and second coextruded tube portions;
Figure 11 is a cross-sectional view of another embodiment of the subject invention wherein the catheter comprises a first member having coextruded tube portions arranged side by side to one another and a guidewire tube coaxial with one of the coextruded tube portions.
Figure 11A is an enlarged partial cross-sectional view of the assembly illustrated in Figure 11;
Figure 12 is a cross-sectional view similar to that illustrated in Figure 11 except that instead of having a coaxial guidewire tube the assembly includes a coaxial guidewire disposed in one of the coextruded tube portions;
Figure 12A is an enlarged partial cross-sectional view of the assembly illustrated in Figure 12.

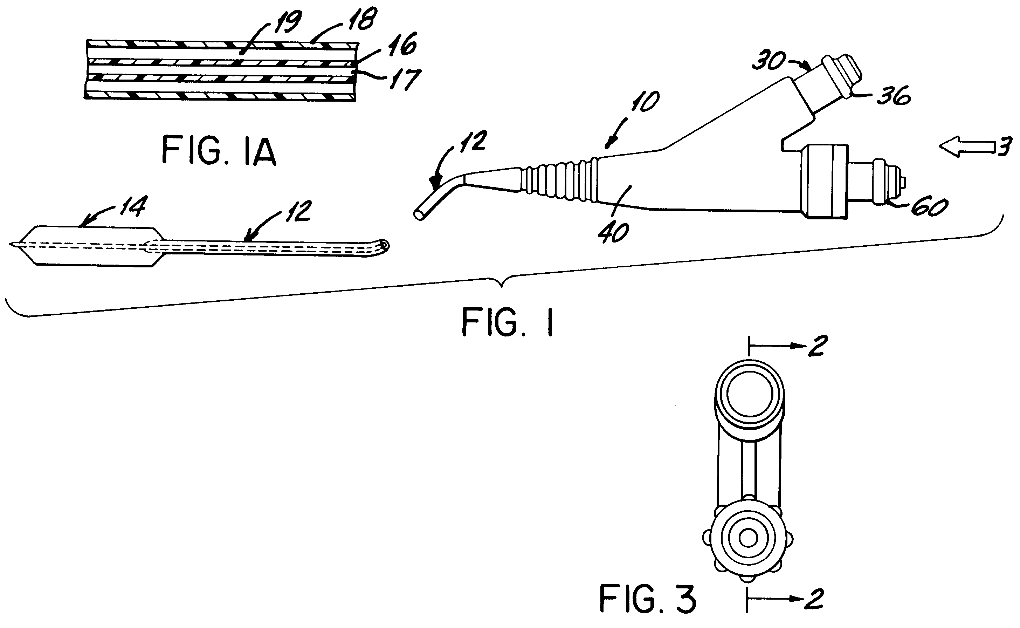

Referring now to Figure 1, there is shown a catheter manifold assembly of the present invention, indicated generally at 10, connected via a longitudinally extending catheter shaft, indicated generally at 12, to an inflatable balloon indicated generally at 14.

The balloon 14 used with the manifold 10 of this invention can be constructed from various angioplasty balloon designs. In accordance with the invention, the catheter shaft 12 may include coaxial inflation and guidewire tubes. The shaft 12 may also include inflation and guidewire tubes that are arranged side-by-side to one another. For purposes of description and illustration only, however, the balloon 14 is illustrated as forming with the catheter shaft 12 a coaxial balloon dilatation catheter that has a first inner guidewire tube 16 formed from a single inner tube defining a guidewire lumen 17 (Fig. 1A) and a single outer inflation tube 18 formed from a single, surrounding outer tube coaxial with the inner tube, and defining an annular inflation lumen 19 therebetween.

As illustrated in Figure 1, the inner guidewire tube 16 extends through the balloon 14 and is secured to the distal end of the balloon. Although the drawing illustrates the outer inflation tube 18 as extending partially into the balloon 14 and being anchored to the inner guidewire tube within the balloon, the outer tube can 1) extend into the balloon and be anchored in the balloon, 2) extend proximal to the balloon and be anchored in that position, 3) extend into the balloon and not be anchored, and 4) extend proximal to the balloon and not be anchored in that position. In one preferred aspect of the invention, the tubes 16,18 are formed of flexible polymeric material such as polyethylene.

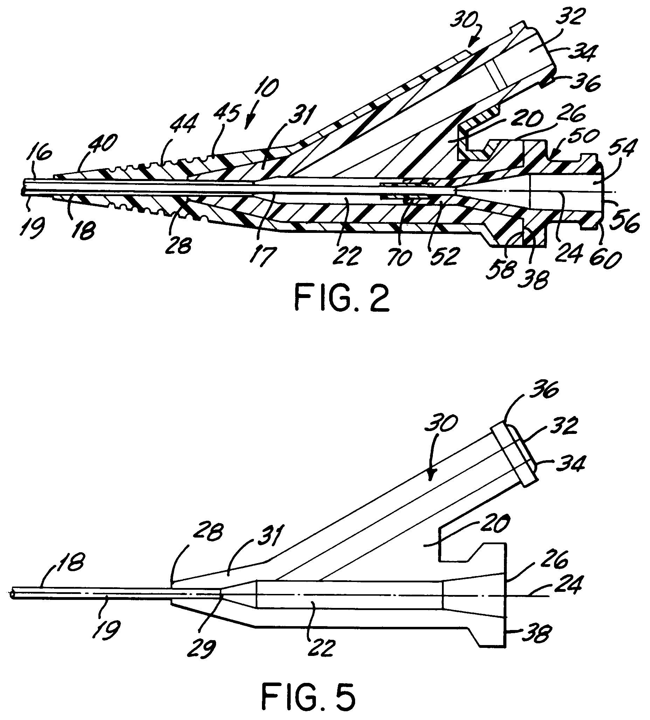

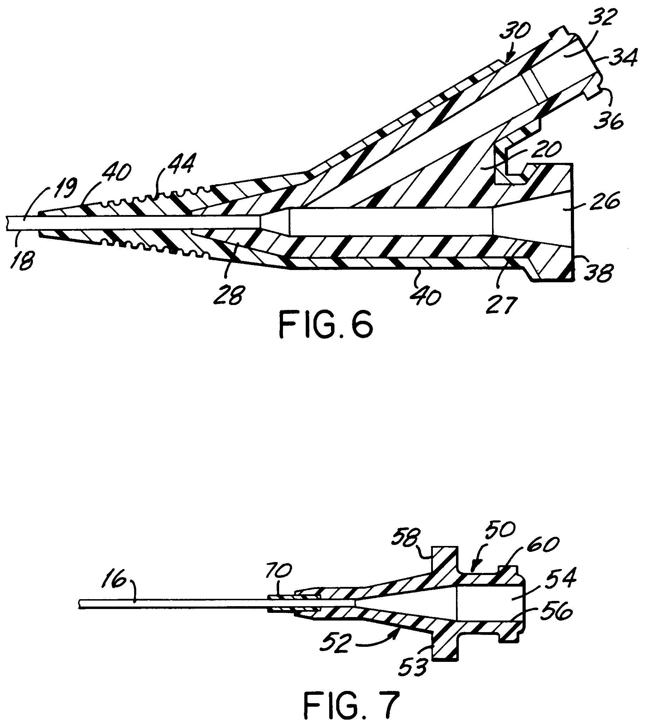

As illustrated in greater detail in Figures 2 and 5, the manifold of the present invention includes a manifold body 20 having a longitudinally extending passage 22 defining a longitudinal axis as illustrated by the centre line 24 extending through the manifold body. The lumen 22 has proximal and distal port openings, 26,28 at respective ends of the manifold body. As illustrated, the distal portion of passage 22 tapers distally to an intermediate point 29; passage 22 has substantially constant cross-section from point 29 to distal port 28. The proximal end of the manifold at the opening 26 forms an annular end face 38.

As illustrated, the manifold body 20 is generally Y-shaped, and includes an obliquely extending inflation leg, indicated generally at 30, having an inflation passage 32 extending from a balloon inflation port 34 at the proximal end of the leg into communication with the inflation lumen 19 of the outer tube (Figure 5). The proximal end of the leg 30 has a connector fitting such as a luer fitting 36 for connecting a conventional inflation-deflation device.

The manifold body is preferably formed of high density polyethylene. To add strength and weldability to the manifold body, a dimensionally stable filler such as microscopic glass spheres approximately 11 microns in size may be added to the high density polyethylene during compounding of the resin. The glass spheres typically can range in size from about 9 to about 13 microns in diameter. The glass spheres preferably are added in amounts of about 17% to 23% by weight, averaging about 20% by weight. The percentage of added glass spheres to plastic resin can range as low as about 10% by weight to as high as about 30% by weight.

In addition, the manifold body may also include a phosphorescent filler which enables the manifold body to glow in the dark.

As illustrated in Figures 2 and 5, the manifold body 20 may be insert molded during manufacture onto the outer inflation tube 18. The term "insert molding", refers to a molding procedure such as injection molding that 1) molds the desired configuration and 2) bonds the outer inflation tube to the manifold body during manifold molding without creating a blockage in the inflation tube. Insert molding has been found the most suitable means for accomplishing the desired bonding between the manifold body and the outer inflation tube.

In accordance with the preferred embodiment of the invention, a flexible polymer strain relief 40 is molded over the distal portion 31 of the manifold body. As illustrated, the strain relief 40 extends over a majority of the surface area of the manifold body including a majority of the surface area of the inflation leg 30. As shown in Figure 2, the strain relief covers most of the leg 30 and manifold body 20. The distal portion 45 of the strain relief tapers distally and extends over the outer inflation tube 18 distally of the distal port opening 28. The distal portion of the strain relief extending distally of the manifold may bend, while supporting the tubes 16,18, thus preventing kinking of the tubes.

The strain relief 40 is formed preferably from an elastomeric plastic material having a hardness ranging from 50 to 100 on the Shore "A" scale. In one embodiment, the strain relief can be formed from a block copolymer composed of polystyrene segments in a matrix of polybutadiene or polyisoprene, known in the trade as KRATON™ "G". Silicone has also been found to be advantageous. The thickness of the strain relief along the manifold body may range from about 0.010 to about 0.042 inches. Although the strain relief 40 could be formed by a non-molding technique, e.g. by dipping the manifold body and outer catheter tube into a solution, it is preferred that the strain relief be insert molded onto the manifold body. Insert molding within an accurately formed mold allows forming a strain relief with the desired, close tolerances.

In the illustrated embodiment, concentric ribs 44 extend around the distal end 45 of the strain relief and provide a gripping or slip-resistant surface with an enhanced ergonomic touch, which is important to a cardiologist during the tense moments normally accompanying the angioplasty procedure. During normal angioplasty procedures, the cardiologist must manipulate the manifold with his fingers to impart the desired results during angioplasty. The physical configuration of the strain relief enhances the cardiologist's touch of the manifold and comfort and ease of manipulation and aids in preventing finger slipping and other detrimental results that may occur from a poor ergonomic "feel" or touch. It also enables the user to receive tactile feedback to assist in locating the catheter in the patient. The ribbed configuration also enhances the bendability or flexibility of the distal portion of the strain relief. In this regard, it is also contemplated that the distal portion of the strain relief may be made of a material having enhanced flexibility, e.g. an elastomeric foam material. It is also contemplated that the strain relief may be formed of a material that enhances slip resistance, e.g. a material with a comparatively high coefficient of friction such as a soft rubber.

As illustrated in Figure 2, a plug cap 50 is received within the proximal end of passage 22 in the manifold body. The plug cap 50 has a throughbore 54 that tapers distally from an enlarged opening 56 at its proximal end. The plug cap 50 includes a distally tapering, generally conical insert member 52, which may be configured for a frictional fit within the distally tapered proximal port portion of passage 22. The plug cap includes an annular shoulder 58 engaged with the annular end face 38 of the manifold body. The plug cap proximal end may be configured as a conventional female luer connector fitting 60 for connecting to male luer locks and other accessory fittings.

As with the manifold body composition, the plug cap 50 can also be formed of high density polyethylene having a dimensionally stable filler material such as a plurality of microscopic glass spheres ranging in size from 9 to 13 microns in diameter mixed therein, averaging 11 microns. The glass spheres add strength to the high density polyethylene to provide a more rigid connector at the proximal end of the plug cap and enhance the weldability of the annular shoulder to the annular end face of the manifold body. The amount by weight of the glass spheres typically varies from 17% to 23%, averaging 20% by weight and can vary from as low as 10% by weight to as high as 30% by weight. Also, as with the manifold body, the plug cap may include a phosphorescent filler.

As illustrated, the plug cap may be molded over the inner guidewire tube 16 such that tube 16 is disposed within the throughbore at the insertion member 52 of the plug cap. The guidewire lumen 17 of the guidewire tube communicates with the throughbore 54 of the plug cap. In accordance with the present invention, plug cap 50 is preferably insert molded by injection molding onto the proximal end of the inner guidewire tube. A support sleeve 70 preferably surrounds inner tube 16 along the overmolded portion of the inner guidewire tube (Figures 2, 7, 8 and 9). Insert molding the plug cap directly onto the inner guidewire tube provides a more secure bond between the end cap and tube that is able to resist the stresses imparted to the bond during normal use and handling. The support sleeve also strengthens the area around the inner guidewire tube adjacent to the distal end of the plug cap.

The manifold is uniquely formed so that the outer inflation tube and inner guidewire tube are bonded securely to the manifold body and plug cap respectively, while providing a strain relief that 1) allows bending and flexing of the tubes adjacent to the manifold distal end 2) provides a comfortable, slip-resistant ergonomic touch that provides tactile feedback, and 3) aids in securing the flexible, polymeric tubes to the manifold assembly.

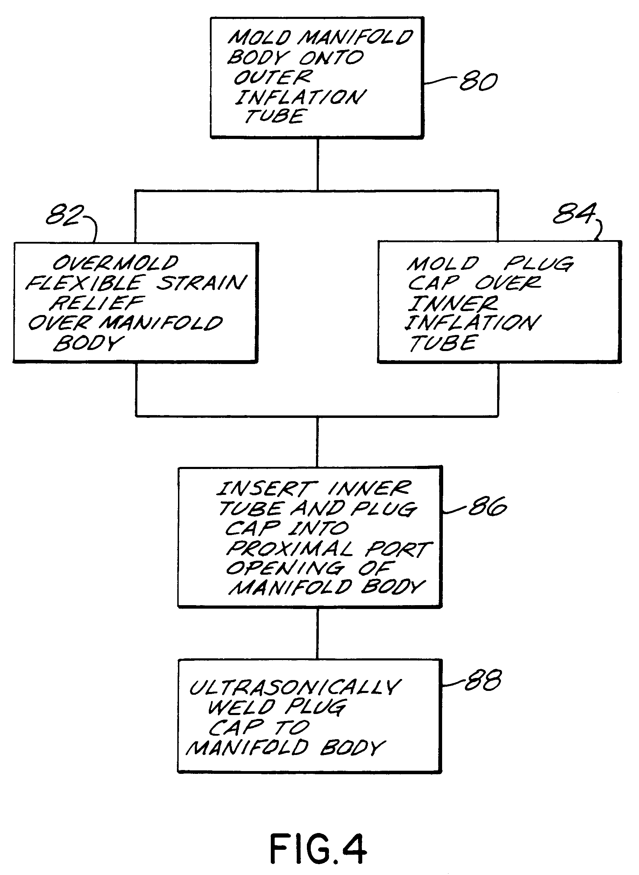

Referring now to Figures 4 to 9, there is illustrated the preferred process of molding the catheter manifold assembly of the present invention. The manifold body 20 may be first formed by insert molding using conventional injection molding techniques. The injection mold is designed so that the outer inflation tube 18 is positioned, like a core, within the injection mold. The injected plastic is formed around the tube. During insert molding, the plastic bonds to the outer surface of the inflation tube 18, forming a strong, thermoplastic bond that resists the tendency of the tube to separate from the manifold body (Figure 5 and Block 80, Figure 4).

In a second molding operation, the molded manifold body 20 is positioned in a second die and the strain relief 40 is insert molded into the die and around the manifold body (Figure 6 and Block 82, Figure 4) to form a strong overmolded mechanical connection between the strain relief and manifold body.

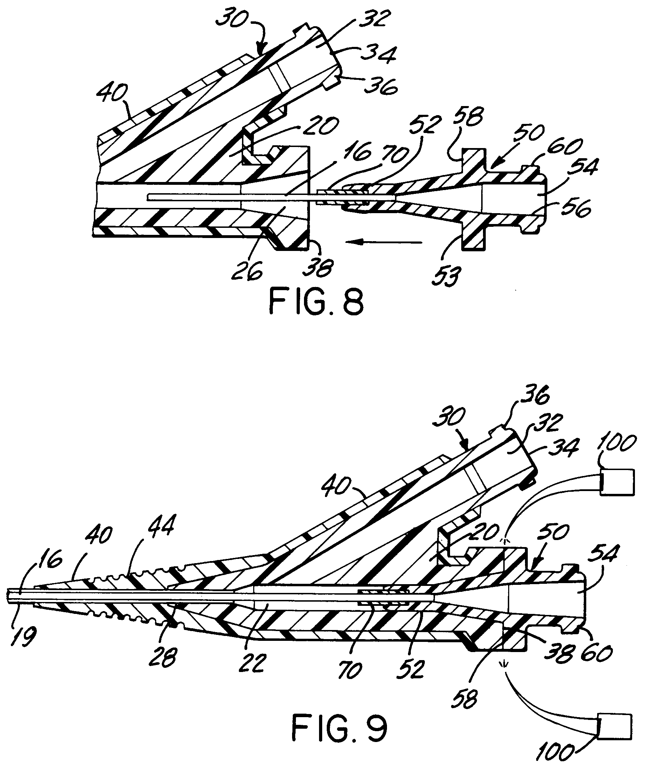

In a parallel, but separate molding step (Figure 4, Block 84, and Figure 7), the inner guidewire tube 16 and support sleeve 70 are positioned in an injection mold and the end cap 50 is molded by flowing molten plastic into the mold and around the inner guidewire tube 16 and support sleeve 70. The formed plug cap 50 having the inner tube bonded thereto is then inserted through the proximal port opening 26 and into passage 22 of the manifold body 20 so that the annular shoulder 58 "bottoms-out" or abuts against the annular proximal end face 38 of the manifold body and the inner tube 16 is disposed within outer tube 18 and through the balloon (Figure 4, Block 86, Figure 8). The plug cap 50 is then ultrasonically welded (Figure 4, Block 88, Figure 9) to the annular end face 38. In this regard, it is preferable that the distal face of annular shoulder 58 include an ultrasonic energy director ring 53 (see Figure 7) which facilitates the ultrasonic welding of the plug cap to the manifold body.

A dimensionally stable filler such as microscopic glass spheres are preferably mixed with the high density polyethylene during compounding of the resin to provide an enhanced structure to the end cap and manifold body allowing a strong weld interface between the annular shoulder and the annular plug cap. In addition, a phosphorescent filler may also be added.

In operation, an inflation-deflation device is attached to the proximal end of the inflation leg 30. A luer lock or other means can be secured onto the proximal end of the plug cap 50 and a guidewire guided into the plug cap, through the inner guidewire tube 16 and through the balloon. A user operates the appropriate inflation-deflation device (or other accessory device) and when necessary, grasps the manifold to manipulate and thereby accomplish the desired therapeutic or diagnostic function.

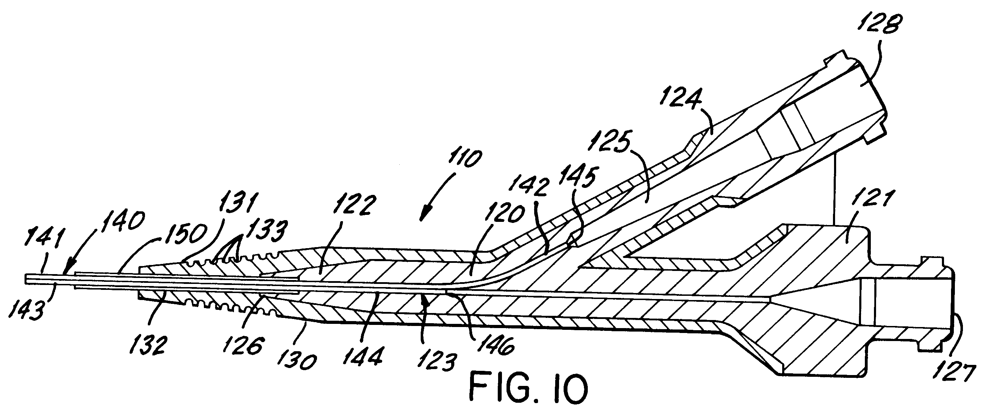

Figure 10 illustrates an embodiment of the subject invention wherein the inflation and guidewire tubes are arranged side-by-side instead of coaxially. As illustrated in Figure 10, the subject assembly 110 includes a manifold body 120 having a proximal end 121 a distal end 122 and a through passage 123 defining the interior surface of the manifold, a proximal port 127 and a distal port 126. Preferably manifold distal end 122 has an outer surface that tapers distally to distal port 126.

As illustrated, the manifold body includes a unitary inflation leg 124 having a leg lumen 125 and a leg port 128.

The catheter 140 includes an inflation tube portion 141 having a proximal end 142 and an inflation lumen 145 which communicates with inflation leg lumen 125 and inflation port 128. Catheter 140 further includes a guidewire tube portion 143 having a proximal end 144 and a guidewire lumen 146 for receiving a guidewire and which communicates with manifold proximal port 127.

Both catheter tube portions 141 and 143 are received in manifold passage 123 and extend distally beyond manifold distal port 126.

A flexible strain relief 130 is molded onto the outer surface of manifold body 120 and around the portion of catheter 140 disposed distal of manifold distal port 126. Preferably, strain relief 130 extends over a majority of the outer surface of manifold body 120, including leg 124. It is also preferable that the distal portion of the strain relief include concentric ribs 133. In accordance with a preferred embodiment of the invention a second tubular strain relief 150 may be directly secured to the catheter 140, a portion of strain relief 150 being disposed in manifold body distal portion 122, in strain relief distal portion 131, and a portion extending distally beyond strain relief 130.

The materials from which the various components of assembly 110 are formed may be the same as those forming the components of the previously described assembly. In addition, the preferred method of manufacturing assembly 110 is substantially the same as the previously described method. In particular, catheter 140 may be formed by extrusion to provide two side by side tube portions 141 and 143. In accordance with the invention, tube portion 143 may be shortened so that the proximal end 142 of tube 141 is longer in the proximal direction that the proximal end 144 of tube portion 143. The manifold body 120 may be molded to include its above described components while also being molded over the proximal ends 142 and 144 of the catheter tube portions. As a result, the proximal end of catheter 140 is firmly secured to the interior of the manifold body. In addition the distal portion of catheter 140 extends distally beyond manifold distal port 126. Strain relief 130 is molded over manifold body 120 and the portion of catheter 140 extending distally beyond manifold distal port 126.

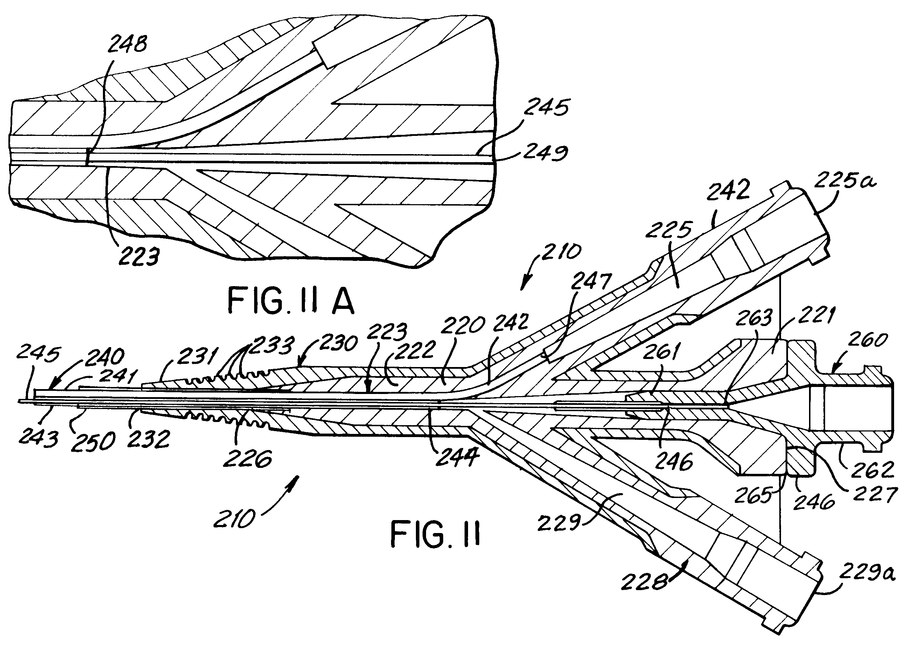

Figures 11 and 11a illustrate another embodiment of the subject manifold distal port 126.

Figures 11 and 11a illustrate another embodiment of the subject invention. As illustrated, the subject assembly 210 includes a manifold body 220 having a proximal end 221, a distal end 222 and a through passage 223 defining the interior surface of the manifold, a proximal port 227 and a distal port 226. Preferably manifold distal end 222 has an outer surface that tapers distally to distal port 226.

As illustrated, the manifold body includes a first unitary inflation leg 224 having a leg lumen 225 and a leg port 225a. The manifold body further includes a second unitary leg 228 having a leg lumen 229 and a leg port 229a.

The catheter 240 includes an inflation tube portion 241 having a proximal end 242 and an inflation lumen 247 which communicates with inflation leg lumen 225 and inflation port 225a. Catheter 240 also includes a fluid tube portion 243 having a proximal end 244 and a fluid lumen 248 which may be used, for example, for passing a fluid supplied through port 229 and a leg lumen 229 which communicates with lumen 248.

As illustrated, catheter tube portions 241 and 243 may be extruded tubes wherein their respective lumens 247 and 248 are disposed side-by-side instead of coaxially. As also illustrated, both catheter tube portions 241 and 243 are received in manifold passage 223 and extend distally beyond manifold distal port 226.

In accordance with this embodiment of the invention, catheter 240 includes a third catheter tube 245 which is disposed within catheter tube 243. Catheter tube 245, which has a lumen 249 for receiving a guidewire, is coaxial with catheter tube 243.

A flexible strain relief 230 is bonded to the outer surface of manifold body 220 and around the portion of catheter 240 disposed including legs 224 and 228. It is also preferable that the distal portion of the strain relief include concentric ribs 233. In accordance with a preferred embodiment of the invention a second tubular strain relief 250 may be directly bonded to the catheter 240, a portion of strain relief 250 being disposed in manifold body distal portion 222, in strain relief distal portion 231, and a portion extending distally beyond strain relief 230.

The subject assembly further includes a plug cap 260 having a distal end 261 a proximal end 262 and an interior bore 263. In accordance with the invention, the proximal end 246 of the guidewire tube is received in plug bore 263 and bonded to the interior of the plug. The plug 260 further includes a shoulder portion 264 having a bonding face 265 which is bonded to the proximal end 221 of the manifold body.

The materials from which the various components of assembly 210 are formed may be the same as those forming the components of the previously described assemblies. In addition, the preferred method of manufacturing assembly 210 is substantially the same as the previously described methods.

In particular, manifold body 220 is molded to have its above described components while also being molded onto the proximal end of catheter 240. Strain relief 230 is molded over the manifold body and the portion of catheter 240 extending beyond manifold distal port 226. Plug 260 is molded to include its above described components while also being molded over the proximal end 246 of the guidewire tube. Such molding securely fixes the plug cap to the guidewire tube. The distal end of the guidewire tube is inserted into the proximal port 227 and passage 223 of the manifold body such that the distal portion 261 of the plug 260 is disposed in passage 223 and bonding face 265 of the plug shoulder abuts against the proximal end 221 of the manifold body. Face 265 may then be secured to the manifold body, for example, by ultrasonic welding.

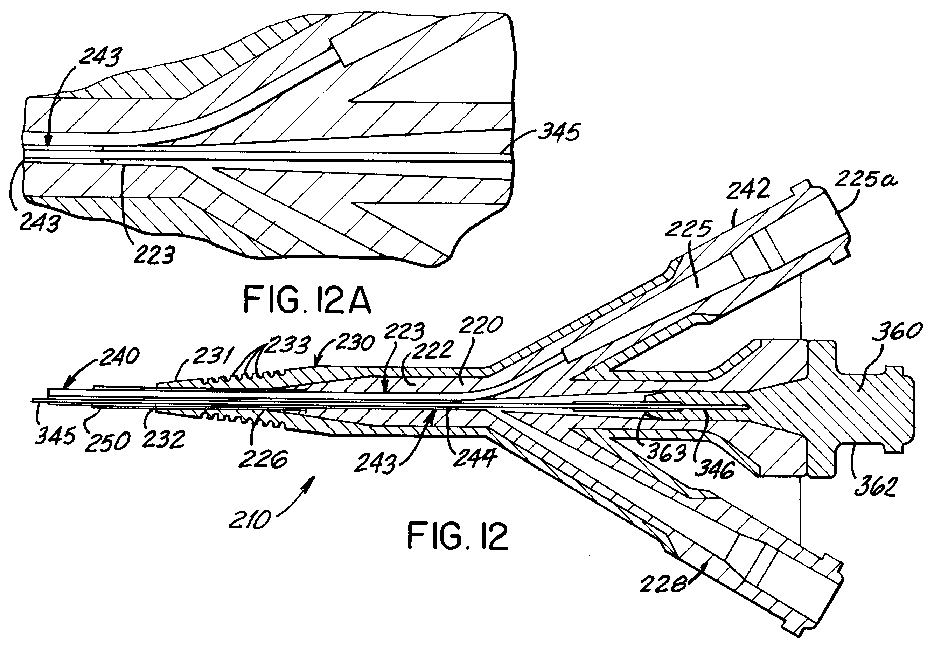

Figures 12 and 12A illustrate a variation on the embodiment of the invention of Figures 11 and 11a. For the sake of simplicity, elements in Figures 12 and 12A which correspond to elements in Figures 11 and 11A bear the same reference numerals. Briefly, the assembly of Figures 12 and 12A differ in that the third catheter element forming catheter 240 in Figures 12 and 12A is a guidewire 345 which is disposed within catheter tube 243 as opposed to a guidewire tube 245 in Figures 11 and 11A. As further illustrated in Figures 11 and 11a, the proximal end 346 of guidewire 345 is received within plug bore 363 and fixed to the interior of the plug in the plug bore 363. It should be noted that whereas plug bore 263 in Figures 11 and 11A is a through bore extending from the distal end of the plug to the proximal end thereof, the proximal end 362 of plug 360 is closed, with bore 363 extending only from the distal end of the plug to an intermediate portion thereof.

The skilled reader should understand that each and every feature disclosed in any or all of the accompanying claims may be combined with any one or all of the said features and that all such combinations of features should be understood to be disclosed by this document.

## Claims

1. A catheter manifold assembly characterised by a manifold body (20) of unitary construction having a proximal end, a distal end, an outer surface, and a longitudinal through passage (22) defining a distal port (28) at said distal end, a proximal port (26) at said proximal end and a manifold inner surface; an elongated first catheter element (18) having a proximal portion disposed within said manifold longitudinal passage and bonded to the inner surface of said manifold body, there being thermoplastic bond portion between said first catheter element (16) and said manifold body (20), the first catheter element extending distally beyond the distal port (28) of the manifold body and having a longitudinal lumen; an elongated second catheter element (16) disposed in said manifold body longitudinal passage and extending distally beyond the distal port of the manifold body, said second catheter element having a proximal end bonded to the inner surface of the manifold body, there being a thermoplastic bond portion between said manifold body and said second catheter element; and a flexible polymer tubular strain relief jacket (40) molded around the outer surface of said manifold body and having a distal portion extending distally beyond the distal end of said manifold body, said distal portion of the strain relief jacket being molded around said first and second catheter elements.

2. An assembly as claimed in claim 1 characterised in that said first and second catheter elements (16,18) are coaxial with each other.

3. An assembly as claimed in claim 1 or claim 2 characterised in that the strain relief jacket (40) extends over a majority of the outer surface of said manifold body (20).

4. An assembly as claimed in any preceding claim characterised in that said manifold body (20) further includes an obliquely extending leg (30) having an outer surface, an interior passage (32) and a proximal end having a leg port (34) communicating with said leg passage and first catheter lumen and wherein said strain relief jacket (40) extends over a majority of the outer surface of said manifold leg.

5. An assembly as claimed in any preceding claim characterised in that said distal portion of the strain relief jacket (40) includes a plurality of concentric ribs (44).

6. An assembly as claimed in any preceding claim characterised in that outer surface of the distal end of said manifold body tapers distally and wherein said distal portion of said strain relief jacket tapers distally.

7. An assembly according to any preceding claim characterised in that said strain relief jacket (50) is formed from a block copolymer of polystyrene segments in a matrix of polybutadiene.

8. An assembly as claimed in any of claims 1-6 characterised in that said strain relief jacket (40) is formed from a block copolymer of polystyrene segments in a matrix of polyisoprene.

9. An assembly as claimed in any preceding claim characterised in that said strain relief jacket (40) is formed from an elastomeric plastic material having a hardness ranging from about 50 to about 100 on the Shore A scale.

10. An assembly as claimed in any preceding claim characterised in that said strain relief jacket (40) is formed from an elastomeric foam material.

11. An assembly according to claim 2 wherein said first catheter element (18) is an inflation tube and said second catheter element is a guidewire tube (16) having a lumen for receiving a guidewire.

12. An assembly as claimed in claim 11 characterised by a plug cap (50) having a distal portion (52) disposed through the proximal port (26) of said manifold body (20) and in the through passage (22) of said manifold body, and a shoulder portion (58) bonded to the proximal end of said manifold body (20), said plug cap (50) further including a bore (54) defining an interior surface of said plug cap, the proximal end of said guidewire tube (16) being received in said plug cap bore and bonded to the interior surface of said plug cap.

13. An assembly as claimed in claim 12 characterised in that said strain relief jacket (40) extends over a majority of the outer surface of said manifold body (20) and said inflation leg (30).

14. A catheter manifold assembly characterised by a manifold body (20) of unitary construction having a proximal end, a distal end, an outer surface and a longitudinal through passage (22) defining a distal port (28) at said distal end, a proximal port (26) at said proximal end and a manifold inner surface, said manifold body further including an obliquely extending inflation leg (30) having an inflation passage (32) communicating with said longitudinal through passage (22) and a proximal end having an inflation port (32); an inflation tube (18) having a proximal portion disposed within said manifold longitudinal passage (22) and bonded to the inner surface of said manifold body (20), the inflation tube extending distally beyond the distal port (28) of the manifold body (20) and having a lumen communicating with the inflation port of said manifold body; a plug member (50) having a distal end, a proximal end and a bore extending from said plug distal end to said plug proximal end, the plug distal end being received through said manifold proximal port and said manifold through passage, said plug member including a shoulder portion (58) bonded to the proximal end of said manifold body (20); a guidewire tube (16) disposed coaxially within said inflation tube (18) in said manifold body longitudinal passage and extending distally beyond the distal port of the manifold body, said guidewire tube having a proximal end bonded to the inner surface of said plug member; and a flexible polymer tubular strain relief jacket (40) bonded around the outer surface of said manifold body (20) and having a distal portion extending distally beyond the distal end of said manifold body, said distal portion of the strain relief jacket (40) being bonded around a portion of said inflation tube (18) extending distally being the distal port of the manifold body (20).

15. An assembly as claimed in claim 14 characterised in that said strain relief jacket (40) is formed from a block copolymer of polystyrene segments in a matrix of polybutadiene.

16. An assembly as claimed in claim 14 characterised in that said strain relief jacket (40) is formed from a block copolymer of polystyrene segments in a matrix of polyisoprene.

17. An assembly as claimed in any of claims 14-16 characterised in that the strain relief jacket (40) has an outer surface having a plurality of concentric ribs.

18. An assembly as claimed in any of claims 14-17 characterised in that the strain relief jacket (40) is made from an elastomeric foam material.

19. A catheter manifold assembly characterised by a manifold body (220) of unitary construction having a proximal end, a distal end, an outer surface and a longitudinal through passage (223) defining a distal port (226) at said distal end, a proximal port (227) at said proximal end and a manifold inner surface, said manifold body (220) further including a first obliquely extending leg (224) having a through passage (225) communicating with said manifold body longitudinal through passage (223) and a proximal end first port (225a) and a second obliquely extending leg (228) having a through passage (229) communicating with the manifold body longitudinal passage (223) and a proximal end having a second port (229a); a first elongated tubular catheter element (241) having a proximal portion disposed within said manifold longitudinal passage and bonded to the inner surface of said manifold body, the first catheter element (241) extending distally beyond the distal port of the manifold body and having a lumen (247) communicating with the first port (225a) of said manifold body; a second elongated tubular catheter element (243) disposed in said manifold body longitudinal passage and extending distally beyond the distal port of the manifold body, said second elongated catheter element (243) having a proximal end bonded to the manifold body inner surface, said second catheter element (243) having a lumen communicating with the through passage of said second leg (228); a plug member (260) having a distal end (261), a proximal end (262) and a bore (263) defining a plug inner surface, the plug distal end being received through said manifold proximal port and said manifold through passage, said plug member including a shoulder (264) portion bonded to the proximal end (221) of said manifold body; a third elongated catheter element (245) coaxially disposed within said second catheter element (243), said third catheter element being received in said plug bore and bonded to the plug inner surface and extending beyond the distal port of the manifold body; and a flexible polymer tubular strain relief jacket (230) secured around the outer surface of said manifold body and having a distal portion extending distally beyond the distal end of said manifold body, said distal portion of the strain relief jacket (40) being secured around a portion of said first and second catheter elements disposed distally of said distal port of said manifold body.

20. An assembly as claimed in claim 19 characterised in that said third catheter element (245) has a lumen communicating with said plug bore.

21. An assembly as claimed in claim 19 characterised in that said third catheter element (245) is a guidewire.

22. A method of forming a catheter manifold assembly comprising the steps of:
providing an elongated catheter member having a proximal end and a distal end, said catheter member including first and second tubular portions each having a proximal end, said first tubular portion defining a first catheter lumen, said second tubular portion defining a second catheter lumen;
molding a manifold body onto the proximal end of said catheter member, said manifold body having a proximal end, a distal end and longitudinal through passage defining a distal port, a proximal port, and a manifold outer surface, the proximal end of said catheter member being disposed within the longitudinal passage of said manifold body, with the proximal ends of said catheter tubular members being bonded to the inner surface of said manifold body as a result of said molding of said manifold body onto said catheter member, said manifold body further including an obliquely extending leg having a leg lumen defining a leg port in communication with said first catheter lumen, said catheter member extending distally beyond the distal port of said manifold body; and
molding a flexible polymer strain relief around the outer surface of said manifold body and around a portion of said catheter member distal of the distal port of said manifold body so as to bond said strain relief to said manifold body and said catheter member and provide a flexible distal strain relief portion disposed distally to said manifold body distal port.

23. A method of forming a catheter manifold assembly comprising the steps of:
providing a first elongated catheter member having a proximal end and a distal end, and defining a first catheter lumen;
molding a manifold body onto the proximal end of said first catheter member, said manifold body having a proximal end, a distal end and longitudinal through passage defining a distal port, a proximal port, and a manifold inner surface, the proximal end of said first catheter member being disposed within the longitudinal passage of said manifold body, so as to bond the proximal end of said first catheter member to the inner surface of said manifold body, said manifold body further including an obliquely extending leg having a leg lumen defining a proximal leg port in communication with said first catheter lumen, said first catheter member extending distally beyond the distal port of said manifold body;
molding a flexible polymer strain relief around the outer surface of said manifold body and around a portion of said first catheter member distal of the distal port of said manifold body so as to bond said strain relief to said manifold body and said first catheter member and provide a flexible distal strain relief portion disposed distal of said manifold body distal port;
providing a second elongated catheter member having a proximal end, a distal end and defining a second catheter lumen;
molding a plug cap member onto the proximal end of said second catheter member, said plug cap member having a distal portion, a proximal portion, and a plug bore defining an inner plug surface, the proximal end of said second catheter member being received in the distal portion of the plug cap through the plug bore and bonded to the inner surface of said plug;
inserting the distal end of said second catheter member through said manifold body proximal port, said longitudinal passage and distally out the manifold body distal port such that the distal portion of said plug member is received in said manifold body longitudinal passage; and
bonding said plug member to the proximal end of said manifold body.

24. A method according to claim 23 wherein said plug member includes a shoulder portion and wherein said step of bonding the plug member to the manifold body comprises the step of bonding the plug shoulder portion to the proximal end of the manifold body.

25. A method according to claim 23 wherein said insertion step further comprises inserting said second catheter member into the proximal end of said first catheter member and the first lumen defined by said first catheter member such that said first and second catheter members are coaxial with one another.

26. A method according to claim 23 wherein said strain relief is molded over a majority of the outer surface of said manifold body.

27. A method according to claim 23 wherein said strain relief is molded over a majority of the outer surface of said leg member.

28. A method according to claim 23 wherein said step of molding the strain relief further comprises the step of molding concentric ribs onto the portion of the strain relief extending distally beyond the distal port of the manifold body.

29. A method according to claim 23 wherein said strain relief is formed from a block copolymer composed of polystyrene segments in a matrix of polybutadiene.

30. A method according to claim 23 wherein said strain relief is formed from a block copolymer composed of polystyrene segments in a matrix of polyisoprene.

31. A method according to claim 23 wherein said strain relief is formed from an elastomeric plastic material having a hardness ranging from 50 to 100 on the Shore A scale.

32. A method according to claim 23 wherein said manifold body is molded from a material formed by mixing a comparatively high density polyethylene and a dimensionally stable filler.

33. A method according to claim 24 wherein the plug shoulder portion is ultrasonically welded to the proximal end of the manifold body.

34. A method according to claim 32 wherein said filler glass spheres comprise glass spheres from about 9 to about 13 microns in size.

35. A method according to claim 33 wherein said glass spheres comprise about 10% to about 30% by weight of the polyethylene.

36. A method of forming a catheter manifold assembly comprising the steps of:
providing a first elongated catheter member having a proximal end and a distal end, said first catheter member including first and second tubular portions each having a proximal end and a distal end, said first tubular portion defining a first catheter lumen, said second tubular portion defining a second catheter lumen;
molding a manifold body onto the proximal end of said first catheter member, said manifold body having a proximal end, a distal end and longitudinal through passage defining a distal port, a proximal port, and a manifold outer surface, the proximal end of said first catheter member being disposed within the longitudinal passage of said manifold body so as to bond the proximal ends of said first catheter tubular members to the inner surface of said manifold body, said manifold body further including an obliquely extending leg having a leg lumen defining a leg port in communication with said first catheter lumen, said first catheter member extending distally beyond the distal port of said manifold body;
providing a second elongated catheter member having a proximal end and a distal end;
molding a flexible polymer strain relief jacket around the outer surface of said manifold body and around a portion of said first catheter member distal of the distal port of said manifold body so as to bond said strain relief jacket to said manifold body and said first catheter member and provide a flexible distal strain relief portion disposed distal of said manifold body distal port;
molding a plug cap member onto the proximal end of said second catheter member, said plug cap member having a distal portion, a proximal portion, and a plug bore defining an inner plug surface, the proximal end of said second catheter member being received in the distal portion of the plug cap through the plug bore and bonded to the inner surface of said plug;
inserting the distal end of said second catheter member through said manifold body proximal port said longitudinal passage and distally out the manifold body distal port such that the distal portion of said plug member is received in said manifold body longitudinal passage; and bonding said plug member to the proximal end of said manifold body.

37. A method according to claim 36 wherein said plug member includes a shoulder portion and wherein said step of bonding the plug member to the manifold body comprises the step of bonding the plug shoulder portion to the proximal end of the manifold body.

38. A method according to claim 36 wherein said insertion step further comprises the step of inserting said second catheter member into the proximal end of the second tubular portion of said first catheter member and the first lumen defined by the second tubular portion said first catheter member such that said second catheter member and the second tubular portion of said first catheter member are coaxial with one another.

39. A method according to claim 38 wherein said second catheter member is a guidewire tube having a lumen for receiving a guidewire.

40. A method according to claim 39 wherein said second catheter member is a guidewire.

41. A method according to claim 36 wherein said step of molding said manifold body includes molding a second leg unitary with said manifold body, said second leg having an outer surface, a leg lumen and a proximal leg port, said leg lumen and leg port of said second leg communicating with said second catheter lumen.

42. A method according to claim 36 wherein said strain relief jacket is molded over a majority of the outer surface of said manifold body.

43. A method according to claim 36 wherein said strain relief jacket is molded over a majority of the outer surface of said leg member.

44. A method according to claim 36 wherein said step of molding the strain relief jacket further comprises the step of molding concentric ribs onto the portion of the strain relief jacket extending distally beyond the distal port of the manifold body.
